(19) Europäisches Patentamt

European Patent Office

Office européen des brevets

(11) **EP 0 664 692 B1**

(12) **EUROPÄISCHE PATENTSCHRIFT**

(45) Veröffentlichungstag und Bekanntmachung des Hinweises auf die Patenterteilung:
**25.10.2000  Patentblatt 2000/43**

(51) Int. Cl.[7]: **A61K 7/06**

(21) Anmeldenummer: **94916934.6**

(22) Anmeldetag: **04.05.1994**

(86) Internationale Anmeldenummer:
**PCT/EP94/01422**

(87) Internationale Veröffentlichungsnummer:
**WO 95/00104 (05.01.1995  Gazette 1995/02)**

(54) **WÄSSRIGES MITTEL ZUR FESTIGUNG VON HAAREN IN FORM EINES HOCHVISKOSEN, VERSPRÜHBAREN GELS**

AQUEOUS HAIR-FIXING AGENT IN THE FORM OF A SPRAYABLE HIGHLY VISCOUS GEL

AGENT AQUEUX DE FIXATION CAPILLAIRE SE PRESENTANT SOUS FORME DE GEL TRES VISQUEUX PULVERISABLE

(84) Benannte Vertragsstaaten:
**DE ES FR GB IT**

(30) Priorität: **23.06.1993 DE 4320797**

(43) Veröffentlichungstag der Anmeldung:
**02.08.1995  Patentblatt 1995/31**

(73) Patentinhaber:
**Wella Aktiengesellschaft**
**64274 Darmstadt (DE)**

(72) Erfinder:
• **KEIL, Wolfgang**
**D-63165 Mühlheim (DE)**
• **SCHMENGER, Jürgen**
**D-64331 Weiterstadt (DE)**
• **STEIN, Bernd**
**D-63768 Hösbach (DE)**

(56) Entgegenhaltungen:
**EP-A- 0 074 264          WO-A-93/00067**
**FR-A- 2 393 573          GB-A- 2 114 580**
**GB-A- 2 134 784          GB-A- 2 222 949**
**US-A- 4 364 837          US-A- 4 971 080**

Bemerkungen:
Die Akte enthält technische Angaben, die nach dem Eingang der Anmeldung eingereicht wurden und die nicht in dieser Patentschrift enthalten sind.

Anmerkung:    Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Gegenstand der vorliegenden Erfindung ist ein wäßriges Mittel zur Festigung von Haaren in Form eines hochviskosen, versprühbaren Gels, welches eine Kombination aus mindestens einem Disaccharid oder einem Disaccharid mit einem Oligosaccharid mit einem kationischen Polymer und einem Verdicker enthält und frei von einwertigen aliphatischen Alkoholen mit 1 bis 4 Kohlenstoffatomen und bestimmten Salzen ist.

Neben der Pflege und der Reinigung der Haare kommt der Formgebung oder Frisurengestaltung in der heutigen Zeit eine wachsende Bedeutung zu, wobei zwischen permanenter und temporärer Formgebung unterschieden wird.

Eine permanenten Verformung der Haare beinhaltet einen chemischen Eingriff in die Haarstruktur mittels eines Dauerwellmittels, während bei einer temporären Formgebung Mittel zur Festigung der Haare zur Anwendung gelangen, deren wesentliche Bestandteile filmbildende Polymere sind.

Filmbildende Polymere werden üblicherweise in alkoholischer oder wäßrig-alkoholischer Lösung in Festigern, Lotionen, Schaumfestigern, Haarsprays und festigenden Gelen eingesetzt.

Das Dokument GB-A-2134784 offenbart beispielsweise ein alkoholfreies Mittel zur Behandlung von Itaaren, welches 1% Maltose, 0,2% eines kationischen Polymers, 0,5% eines Verdichers und 3% anorganische Salze enthält.

Nachteile bekannter Gelformulierungen sind im besonderen Maße die schlechte Dosierbarkeit beziehungsweise Verteilbarkeit dieser Gele, die üblicherweise aus einer Tube entnommen werden, und die damit verbundene größere Belastung auf dem jeweiligen Haar.

Es bestand daher die Aufgabe, ein gelförmiges Mittel zur Festigung der Haare zur Verfügung zu stellen, das eine gute Festigung der Haare bewirkt und sich leicht dosieren und im Haar verteilen läßt.

Überraschenderweise wurde nunmehr gefunden, daß durch ein wäßriges Mittel zur Festigung der Haare in Form eines versprühbaren Gels mit einer Viskosität von 200 - 10.000 mPa • s, welches eine Kombination eines Disaccharids oder einer Mischung aus Disaccharid und Oligosaccharid mit einem kationischen Polymer und einem Verdicker enthält und das frei von einwertigen aliphatischen Alkoholen mit 1 bis 4 Kohlenstoffatomen und bestimmten anorganischen Salzen ist, die gestellte Aufgabe in hervorragender Weise gelöst wird.

Gegenstand der vorliegenden Erfindung ist daher ein wäßriges Mittel zur Festigung von Haaren in Form eines hochviskosen, versprühbaren Gels, mit einem Gehalt an

a) 0,1 bis 50 Gewichtsprozent mindestens eines Disaccharids oder einer Mischung aus 2 bis 37 Gewichtsprozent mindestens eines Disaccharids und 63 bis 98 Gewichtsprozent mindestens einem Oligosaccharids,

b) 0,05 bis 30 Gewichtsprozent mindestens eines kationischen Polymers und

c) 0,05 bis 10 Gewichtsprozent mindestens eines Verdickers,

welches

d) frei von einwertigen aliphatischen Akoholen mit 1 bis 4 Kohlenstoffatomen ist und

e) keine anorganischen Salze von Alkalimetallen oder anorganische Salze von zweiwertigen oder dreiwertigen Metallen enthält.

Das erfindungsgemäße versprühbare Gel weist vorzugsweise eine Viskosität von 500 bis 6.000 mPa • s (Millipascalsekunden) auf. Die Viskosität des erfindungsgemäßen Gels wurde mit einem Haake-Viskositätsmeßgerät VT 501 bei 25 Grad Celsius und einem SV-DIN-Schergefälle von 12,9 $s^{-1}$ (Reihe B, Stellung 5) gemessen.

Das erfindungsgemäße Mittel ist, obwohl es als hochviskoses Gel vorliegt, mit einer üblichen mechanischen Sprühvorrichtung (zum Beispiel einer Sprühpumpe) oder mit einem Treibmittel aus einem üblichen Aerosol-Druckbehälter mit einem üblichen Sprühkopf als feinverteiltes Gel versprühbar.

Durch seine Versprühbarkeit kann das erfindungsgemäße Mittel genau dosiert und gleichmäßig auf dem Haar verteilt werden. Der durch das erfindungsgemäße Mittel auf dem Haar erzeugte Polymerfilm ist elastisch und beständig gegenüber Luftfeuchtigkeit.

Das erfindungsgemäße Mittel weist eine sehr gute Festigungswirkung auf und ist leicht auswaschbar. Trotz seines Saccharidgehalts fühlt sich das mit dem erfindungsgemäßen Mittel behandelte Haar überraschenderweise nicht klebrig an. Darüber hinaus ist das erfindungsgemäße Mittel frei von den üblicherweise in Mitteln zur Festigung der Haare enthaltenen einwertigen aliphatischen Alkoholen mit 1 bis 4 Kohlenstoffatomen.

Saccharidhaltige Haarbehandlungsmittel sind beispielsweise aus der DE-OS 34 04 627 bekannt. Die beschriebenen Mittel liegen bevorzugt als Lotionen vor und enthalten zwingend Salze. Ein Hinweis auf die Zurverfügungstellung eines Mittels zur Festigung der Haare in Form eines hochviskosen, versprühbaren Gels, das sich gut dosieren und leicht und gleichmäßig verteilen läßt, ist der DE-OS 34 04 627 nicht zu entnehmen.

Unter Oligosacchariden werden in der vorliegenden Anmeldung Saccharide mit 3 bis 6 acetalartig miteinander verbundenen Monosacchariden, verstanden.

Von den Mischungen von Disaccharid und Oligosaccharid, die als Komponente a) in dem erfindungsgemäßen Mittel enthalten sein können, sind Mischungen von Glucosedisacchariden und Glucoseoligosacchariden bevorzugt, die beispielsweise von der Firma Cerestar, Krefeld/BRD unter der Handelsbezeichnung C-Pur® vertrieben werden. Besonders bevorzugt enthält das erfindungsgemäße Mittel als Komponente a) Maltose oder die unter der Handelsbezeichnung C-Pur® 01924 von der Firma Cerestar, Krefeld/BRD vertriebene Glucosedisaccharid-Glucoseoligosaccharid-Mischung.

Das erfindungsgemäße Mittel enthält die Komponente a) bevorzugt in einer Menge von 1 bis 25 Gewichtsprozent.

Von den kationischen Polymeren, die als Komponente b) in dem erfindungsgemäßen Mittel enthalten sind, sind Polyvinylpyrrolidon/Dimethylaminoethylmethacrylat-Polymere bevorzugt. Besonders bevorzugt enthält das erfindungsgemäße Mittel das unter der Handelsbezeichnung Gafquat® 755 N von der Firma Gaf Co., New York/USA vertriebene Polyvinylpyrrolidon/Diaminoethylmethacrylat-Copolymer. Weitere kationische Polymere sind beispielsweise das von der Firma BASF AG, Ludwigshafen/BRD unter dem Handelsnamen Luviquat® H11 550 vertriebene Copolymer aus Polyvinylpyrrolidon und Imidazoliminmethochlorid, das von der Firma Calgon, Pittsburgh/USA unter dem Handelsnamen Merquat® Plus 3330 vertriebene Terpolymer aus Dimethyldiollylammoniumchlorid, Natriumacrylat und Acrylamid, das von der Firma ISP/USA unter dem Handelsnamen Gaffix® VC 713 vertriebene Terpolymer aus Vinylpyrrolidon, Dimethylaminoethylmethacrylat und Vinylcaprolactam, das von der Firma Amerchol, Edison/USA, unter dem Handelsnamen Polymer IR® vertriebene quaternierte Ammoniumsalz der Hydroxyethylcellulose und einem trimethylammonium-substituierten Epoxid, das von der Firma Gaf unter dem Handelsnamen Gafquat® HS 100 vertriebene Vinylpyrrolidon/Methacrylamidopropyltrimethylammoniumchlorid-Copolymer und das von der Firma Goldschmidt, Essen/BRD unter dem Handelsnamen Abil® Quat 3272 vertriebene diquaternäre Polydimethylsiloxan.

Das erfindungsgemäße Mittel enthält die Komponente b) bevorzugt in einer Menge von 0,05 bis 10 Gewichtsprozent.

Von den Verdickern die als Komponente c) in dem erfindungsgemäßen Mittel enthalten sind, sind Homopolymere der Acrylsäure mit einem Molekulargewicht von 2.000.000 bis 6.000.000, die beispielsweise von der Firma BF Goodrich, Cleveland/USA unter der Handeisbezeichnung Carbopol® vertrieben werden, bevorzugt. Besonders bevorzugt enthält das erfindungsgemäße Mittel ein Acrylsäurehomopolymer mit einem Molekulargewicht von 4.000.000, das beispielsweise von der Firma BF Goodrich unter der Handelsbezeichnung Carbopol® 940 vertrieben wird. Weitere Verdicker sind beispielsweise die von der Firma BF Goodrich unter dem Handelsnamen Carbopol® ETD 2001 oder von der Firma Protex/Frankreich unter dem Handelsnamen Modarez V 600 PX vertriebene Acrylsäurehomopolymere, das von der Firma Hoechst/BRD unter dem Handelsnamen Hostacerin® PN 73 vertriebene Polymer aus Acrylsäure und Acrylamid (Natriumsalz) mit einem Molekulargewicht von 2.000.000 bis 6.000.000 und das von der Firma Alban Muller, Montreuil/Frankreich unter dem Handelsnamen Amigel® vertriebene Sclerotium Gum.

Das erfindungsgemäße Mittel enthält bevorzugt 0,1 bis 5 Gewichtsprozent der Komponente c).

Das erfindungsgemäße Mittel kann zusätzlich 0,1 bis 15 Gewichtsprozent, bevorzugt 0,1 bis 7 Gewichtsprozent mindestens eines nicht-ionischen Polymers enthalten. Von den nicht-ionischen Polymeren, die in dem erfindungsgemäßen Mittel enthalten sein können, sind Polyvinylpyrrolidon-Polymere bevorzugt, die beispielsweise von der Firma BASF AG, Ludwigshafen/BRD unter der Handelsbezeichnung Luviskol® vertrieben werden. Von den Polyvinylpyrrolidonpolymeren kann jenes besonders bevorzugt in dem erfindungsgemäßen Mittel enthalten sein, das unter der Handelsbezeichnung Luviskol® VA 64 von der BASF AG vertrieben wird.

Als ein nicht-ionisches Polymer natürlichen Ursprungs ist beispielsweise Hydroxypropylchitosan zu nennen.

Das erfindungsgemäße Mittel kann zusätzlich 0,1 bis 15 Gewichtsprozent, bevorzugt 0,1 bis 7 Gewichtsprozent mindestens eines anionischen Polymers enthalten. Von den anionischen Polymeren, die in dem erfindungsgemäßen Mittel enthalten sein können, sind insbesondere das von der Firma National Starch, Braunston/Großbritannien unter dem Handelsnamen Flexan® 130 vertriebene Natriumpolystyrolsulfonat und das von der Firma Sandoz, Basel/Schweiz unter dem Handelsnamen Diahold® A 503 vertriebene Copolymer aus Methacrylsäure und Methacrylat zu nennen. Weitere anionische Polymere sind das von der Firma Stephan, Northfield/USA, unter dem Handelsnamen Stephanhold® R-1 vertriebene Terpolymer aus Polyvinylpyrrolidon, Ethylmethacrylat und Methacrylsäure, das von der Firma Sandoz unter dem Handelsnamen Diaformer® Z 301 vertriebene Copolymer aus Methacryloylethylbetain und Methacrylat und Schellack, beispielsweise Schellack MHP 210 der Firma Pennig/BRD.

Das erfindungsgemäße Mittel ist vorzugsweise frei von organischen Lösungsmitteln.

Das erfindungsgemäße Mittel weist einen Wassergehalt von 20 bis 98 Gewichtsprozent auf, wobei ein Wassergehalt von 50 bis 96 Gewichtsprozent bevorzugt ist. Das erfindungsgemäße Mittel weist einen pH-Wert von 4 bis 9, vorzugsweise von 6 bis 8, auf.

Selbstverständlich kann das erfindungsgemäße Mittel neben den genannten Bestandteilen auch übliche kosmetische Zusätze, beispielsweise Parfümöle in einer Menge von etwa 0,1 bis 5,0 Gewichtsprozent; Trübungsmittel, wie zum Beispiel Ethylenglykoldistearat, in einer Menge von etwa 0,1 bis 5,0 Gewichtsprozent; Perlglanzmittel, wie zum Beispiel ein Gemisch aus Fettsäuremonoalkylolamid und Ethylenglykoldistearat, in einer Menge von 0,01 bis 10,0 Gewichtsprozent; zur Neutralisierung geeignete Amine oder Alkanolamine, wie zum Beispiel 2-Amino-2-methyl-propanol, Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide, Glyceridalkoxylate, wie zum Beispiel die Ester der hydrierten Rizinusölfettsäuren mit oxethyliertem Glycerin, in einer Menge von 0,01 bis 15 Gewichtsprozent; sowie Farbstoffe, wie zum Beispiel Fluorescein-Natriumsalz, in einer Menge von etwa 0,01 bis 1,0 Gewichtsprozent; weiterhin haarpflegende Zusätze, wie zum Beispiel Pantothensäure in einer Menge von etwa 0,01 bis 10 Gewichtsprozent sowie ferner Feuchthaltemittel, wie 1,2-Propandiol und Glycerin, Farbstoffe, Lichtschutzmittel, Antioxidantien, Glanzgeber, wie zum Beispiel Sorbitol, und Konservierungsstoffe, wie zum Beispiel p-Hydroxybenzoesäureester, Sorbinsäure, Salicylsäure, Mandelsäure oder Ameisensäure, in einer Menge von 0,01 bis 10 Gewichtsprozent.

Das erfindungsgemäße Mittel zur Festigung der Haare kann gegebenenfalls durch einen Gehalt an kosmetischen Farbstoffen das Haar gleichzeitig färben oder tönen. Derartige Präparate sind unter anderem als Farbfestiger oder Tönungsfestiger im Handel bekannt. Sie enthalten zusätzlich übliche direkt auf das Haar aufziehende Farbstoffe, beispielsweise aromatische Nitrofarbstoffe, zum Beispiel 1,4-Diamino-2-nitro-benzol, Pikraminsäure, 1-N-Hydroxyethylamino-4-mehtyl-2-nitrobenzol, 1-Hydroxy-2-amino-4-nitrobenzol und 1,4-bis(2-Hydroxethyl)amino-2-nitro-5-chlorbenzol, Azofarbstoffe, zum Beispiel Acid Brown 4 (C.I. 14 805), Anthrachinonfarbstoffe, zum Beispiel Disperse Violet 4 (C.I. 61 105) und Triphenylmethanfarbstoffe, zum Beispiel Basic Violet 1 (C.I. 42 535), Basic Violet 14 (C.I. 42 510) , Basic Red 76 (C.I. 12 245) oder Basic Blue 7 (C.I. 42 595 : 1), wobei die Farbstoffe dieser Klassen, je nach Art ihrer Substituenten, sauren, nichtionogenen oder basischen Charakter haben können. Ihre Gesamtkonzentration in dem erfindungsgemäßen Mittel beträgt üblicherweise etwa 0,01 bis 2,0 Gewichtsprozent.

Das erfindungsgemäße Mittel zur Festigung der Haare wird vorzugsweise unter Verwendung eines Treibmittels oder mit Hilfe einer mechanisch betriebenen Sprühvorrichtung versprüht.

Wenn das erfindungsgemäße Mittel mit Hilfe eines Treibmittels versprüht wird, so enthält es bevorzugt 25 bis 75 Gewichtsprozent eines Treibmittels und wird in einen Druckbehälter abgefüllt.

Als Treibmittel sind beispielsweise niedere Alkane, wie zum Beispiel n-Butan, i-Butan und Propan, oder auch deren Gemische mit Dimethylether sowie ferner bei den in Betracht kommenden Drücken gasförmig vorliegende Treibmittel, wie beispielsweise $N_2$, $N_2O$ und $CO_2$, sowie Gemische der vorstehend genannten Treibmittel geeignet.

Das erfindungsgemäße Mittel zur Festigung der Haare wird besonders bevorzugt mit Hilfe einer geeigneten mechanisch betriebenen Sprühvorrichtung versprüht.

Unter mechanischen Sprühvorrichtungen sind solche Vorrichtungen zu verstehen, welche das Versprühen einer Flüssigkeit ohne Verwendung eines Treibmittels ermöglichen. Als geeignete mechanische Sprühvorrichtung kann beispielsweise eine Sprühpumpe oder ein mit einem Sprühventil versehener elastischer Behälter, in den das erfindungsgemäße kosmetische Mittel unter Druck abgefüllt wird, wobei sich der elastische Behälter ausdehnt und aus dem das Mittel infolge der Kontraktion des elastischen Behälters bei Öffnen des Sprühventils kontinuierlich abgegeben wird, verwendet werden.

Das erfindungsgemäße Mittel zur Festigung der Haare bewirkt eine ausgezeichnete Festigung ohne einen klebrigen Griff zu erzeugen, ist gut aus dem Haar ausbürstbar und leicht aus dem Haar auswaschbar. Da es mit Hilfe mechanischer Sprühvorrichtungen oder unter Verwendung von Treibgasen versprühbar ist, läßt es sich leicht dosieren und gut im Haar verteilen.

Die nachfolgenden Beispiele sollen den Gegenstand der Erfindung näher erläutern:

**Beispiele**

**Beispiel 1: Gel-Spray mit normaler Festigung**

| 6,00 g | Maltose |
|---|---|
| 0,06 g | Polyvinylpyrrolidon/Dimethylaminoethylmethacry lat-Copolymer (Gafquat® 755 N der Firma Gaf Co./USA) |
| 0,20 g | Acrylsäurehomopolymer mit einem Molekulargewicht von 4.000.000 (Carbopol® 940 der Firma BF Goodrich/USA) |
| 0,23 g | 2-Amino-2-methyl-propanol |
| 0,10 g | Parfümöl |
| 93,41 g | Wasser |
| 100,00 g | |

**Beispiel 2: Gel-Spray mit starker Festigung**

| 8,00 g | einer Mischung aus Glucosedisaccharid |

| | |
|---|---|
| | und Glucoseoligosaccharid (C-Pur® 01924 der Firma Cerestar/BRD) |
| 0,08 g | Polyvinylpyrrolidon/Dimethylaminoethylmethacry lat-Copolymer (Gafquat® 755 N der Firma Gaf Co./USA) |
| 0,22 g | Acrylsäurehomopolymer mit einem Molekulargewicht von 4.000.000 (Carbopol® 940 der Firma BF Goodrich/USA) |
| 0,26 g | 2-Amino-2-methyl-propanol |
| 0,15 g | Parfümöl |
| 91,29 g | Wasser |
| 100,00 g | |

**Beispiel 3: Gel-Spray mit extra starker Festigung**

| | |
|---|---|
| 10,00 g | einer Mischung aus Glucosedisaccharid und Glucoseoligosaccharid (C-Pur® 01924 der Firma Cerestar/BRD) |
| 0,50 g | Dimethyldiallylammoniumchlorid/Natriumacrylat/ Acrylamid-Terpolymer (Merquat® Plus 3 330 der Firma Calgon/USA) |
| 0,25 g | Copolymer aus Acrylsäure und Acrylamid-Natriumsalz (Hostacerin® PN 73 der Firma Hoechst/BRD). |
| 3,00 g | Polyvinylpyrrolidon (Luviskol® VA 64 der Firma BASF AG/BRD) |
| 0,20 g | mit 14 mol Ethylenoxid oxethyliertes Nonylphenol |
| 0,20 g | para-Hydroxybenzoesäure-Methylester |
| 0,10 g | Parfümöl |
| 85,75 g | Wasser |
| 100,00 g | |

**Beispiel 4: Gel-Spray mit normaler Festigung**

| | |
|---|---|
| 5,00 g | einer Mischung aus Glucosedisaccharid und Glucoseoligosaccharid (C-Pur® 01924 der Firma Cerestar/BRD) |
| 0,50 g | Diquaternäres Polydimethylsiloxan (Abil® Quat 3272 der Firma Goldschmidt/BRD) |
| 1,00 g | Sclerotium-Gum (Amigel® der Firma Alban Muller |
| 0,20 g | mit 14 mol Ethylenoxid oxethyliertes Nonylphenol |
| 0,20 g | para-Hydroxybenzoesäuremethylester |
| 0,10 g | Parfümöl |
| 93,00 g | Wasser |
| 100,00 g | |

**Beispiel 5: Gel-Spray mit normaler Festigung**

| | |
|---|---|
| 3,00 g | Maltose |

| | |
|---|---|
| 0,50 g | Polyvinylpyrrolidon/Imidazoliminmethochlorid (Luviquat® HM 550 der Firma BASF AG/BRD) |
| 0,20 g | Acrylsäurehomopolymer mit einem Molekulargewicht von 4 000 000 (Carbopol® 980 der Firma BF Goodrich/USA) |
| 0,25 g | Ammoniak, 25 prozentige, wäßrige Lösung |
| 0,50 g | Schellack |
| 0,20 g | mit 14 mol Ethylenoxid oxethyliertes Nonylphenol |
| 0,20 g | para Hydroxybenzoesäuremethylester |
| 0,10 g | Parfümöl |
| 95,05 g | Wasser |
| 100,00 g | |

**Beispiel 6: Wet Gel**

| | |
|---|---|
| 3,00 g | einer Mischung aus Glucosedisaccharid und Glucoseoligosaccharid (C-Pur® 01924 der Firma Cerestar/BRD) |
| 0,50 g | Vinylpyrrolidon/Dimethylaminoethylmethacrylat/ Vinylcaprolactam-Terpolymer (Gaffix® VC 713 der Firma ISP/USA) |
| 0,25 g | Acrylsäurehomopolymer mit einem Molekulargewicht von 6 000 000 (Modarez® V 600 PX der Firma Protex/Frankreich) |
| 0,20 g | 2-Amino-2-methylpropanol |
| 0,20 g | mit 14 mol Ethylenoxid oxethyliertes Nonylphenol |
| 0,10 g | Parfümöl |
| 95,75 g | Wasser |
| 100,00 g | |

**Beispiel 7: Gel-Spray mit starker Festigung**

| | |
|---|---|
| 3,00 g | Maltose |
| 0,50 g | Vinylpyrrolidon/Methacrylamidopropyltrimethylammoniumchlorid-Copolymer (Gafquat® HS 100 der Firma Gaf/USA) |
| 0,20 g | Acrylsäurehomopolymer mit einem Molekulargewicht von 4 000 000 (Carbopol® 980 der Firma BF Goodrich/USA) |
| 1,00 g | Methacrylsäure/Methacrylat-Copolymer (Diahold® A 503) der Firma Sandoz/Schweiz) |
| 0,50 g | Hydroxypropylchitosan |
| 0,20 g | mit 14 mol Ethylenoxid oxethyliertes Nonylphenol |
| 0,10 g | Parfümöl |
| 94.50 g | Wasser |
| 100,00 g | |

Das Mittel wird mit wäßriger Natronlauge auf pH 7,0 eingestellt.

**Beispiel 8:**

| | |
|---|---|
| 7,000 g | einer Mischung aus Glucosedisaccharid und Glucoseoligosaccharid (C-Pur® 01924 der Firma Cerestar/BRD) |
| 0,500 g | Polyvinylpyrrolidon/Dimethylaminoethylmethacrylat-Copolymer (Gafquat® 755 N der Firma Gaf Co./USA) |
| 0,250 g | Acrylsäurehomopolymer mit einem Molekulargewicht von 4.000.000 (Carbopol® 980 der Firma BF Goodrich/USA) |
| 0,290 g | 2-Amino-2-methyl-propanol |
| 0,200 g | mit 45 mol Ethylenoxid oxethyliertes hydriertes Rizinusöl |
| 0,200 g | mit 9 mol Ethylenoxid und 2 mol Prophylenoxid akoxylierter Cetylstearylalkohol |
| 1,800 g | para-Hydroxybenzoesäuremethylester |
| 0,180 g | Basic Red 76 (C.I. 12 245) |
| 0,015 g | Basic Violet 14 (C.I. 42 510) |
| 0,230 g | 1-N-Hydroxyethylamino-4-methyl-2-nitro-benzol |
| 0,300 g | 1,4-bis (2-Hydroxyethyl)amino-2-nitro-5-chlorbenzol |
| 0,200 g | Parfümöl |
| 88,835 g | Wasser |
| 100,000 g | |

**Beispiel 9:**

| | |
|---|---|
| 7,0000 g | einer Mischung aus Glucosedisaccharid und Glucoseoligosaccharid (C-Pur® 01924 der Firma Cerestar/BRD) |
| 0,5000 g | Polyvinylpyrrolidon/Dimethylaminoethylmethacrylat-Copolymer (Gafquat® 775 N der Firma Gaf Co./USA) |
| 0,2500 g | Acrylsäurehomopolymer mit einem Molekulargewicht von 4.000.000 (Carbopol® 980 der Firma BF Goodrich/USA) |
| 0,2900 g | 2-Amino-2-methyl-propanol |
| 0,2000 g | mit 45 mol Ethylendioxid oxethyliertes hydriertes Rizinusöl |
| 0,2000 g | mit 9 mol Ethylendioxid und 2 mol Propylenoxid alkoxylierter Cetylstearylalkohol |
| 1,8000 g | para-Hydroxybenzoesäuremethylester |
| 0,0300 g | 1-N-Hydroxyethylamino-4-methyl-2-nitro-benzol benzol |
| 0,0035 g | 1,4-bis(2-Hydroxyethyl)amino-2-nitro-5-chlorbenzol |
| 0,2000 g | Parfümöl |
| 89,5265 g | Wasser |
| 100,0000 g | |

Alle Prozentangaben in der vorliegenden Anmeldung stellen Gewichtsprozente dar.

**Patentansprüche**

1. Wäßriges Mittel zur Festigung von Haaren in Form eines versprühbaren Gels mit einer Viscosität von 200 - 10.000 mPa • s, gemessen mit einen Haake-Viskositäts meßgerät VT 501 bei 25°C und einem SV-DIN Schergefälle von 12,9 $s^{-1}$, Reihe B, Stellung 5, mit einem Gehalt an

   a) 0,1 bis 50 Gewichtsprozent mindestens eines Disaccharids oder einer Mischung aus 2 bis 37 Gewichtsprozent mindestens eines Disaccharids und 63 bis 98 Gewichtsprozent mindestens eines Oligosaccharids,

   b) 0,05 bis 30 Gewichtsprozent mindestens eines kationischen Polymers

   c) 0,05 bis 10 Gewichtsprozent mindestens eines Verdickers,
   welches

   d) frei von einwertigen aliphatischen Alkoholen mit 1 bis 4 Kohlenstoffatomen ist
   und

   e) keine anorganischen Salze von Alkalimetallen, oder anorganische Salze von zweiwertigen oder dreiwertigen Metallen enthält.

2. Mittel nach Anspruch 1, dadurch gekennzeichnet, daß es frei von organischen Lösungsmitteln ist,

3. Mittel nach Anspruch 1, dadurch gekennzeichnet, daß es als Komponente a) Mischungen von Glucosedisacchariden und Glucoseoligosacchariden enthält.

4. Mittel nach Anspruch 3, dadurch gekennzeichnet, daß es als Komponente a) Maltose enthält.

5. Mittel nach einem der Ansprüche 1 bis 4, dadurch gekennzeichnet, daß es 1 bis 25 Gewichtsprozent der Komponente a) enthält.

6. Mittel nach einem der Ansprüche 1 bis 5, dadurch gekennzeichnet, daß es als Komponente b) ein Polyvinylpyrrolidon/Dimetyhlaminoethylmethacrylat-Polymer, ein Polyvinylpyrrolidon/Imidazolimin-methochloridCopolymer, ein Dimethyldiallyl-ammoniumchlorid/Natriumacrylat/Acrylamid-Terpolymer, ein Vinylpyrrolidon/Dimethylaminoethylmethacrylat/Vinylcaprolactam-Terpolymer, ein quaternisiertes Ammoniumsalz oder Hydroxyethylcellulose und einem Trimethylammonium substituierten Epoxid, ein Vinylpyrrolidon/Methacrylamidopropyltrimethylammonium-Copolymer oder ein diquaternäres Polydimethylsiloxan enthält.

**7.** Mittel nach einem der Ansprüche 1 bis 6, dadurch gekennzeichnet, daß es 0,05 bis 10 Gewichtsprozent der Komponente b) enthält.

**8.** Mittel nach einem der Ansprüche 1 bis 7, dadurch gekennzeichnet, daß es als Komponente c) ein Homopolymer der Acrylsäure, ein Acrylsäure/Acrylamid-Copolymer oder ein Sclerotium-Gum enthält.

**9.** Mittel nach Anspruch 8, dadurch gekennzeichnet, daß es ein Acrylsäurehomopolymer mit einem mittleren Molekulargewicht von 4.000.000 enthält.

**10.** Mittel nach Anspruch 8, dadurch gekennzeichnet, daß es ein Acrylsäurehomopolymer mit einem mittleren Molekulargewicht von 2.000.000 bis 6.000.000 enthält.

**11.** Mittel nach einem der Ansprüche 1 bis 10, dadurch gekennzeichnet, daß es 0,1 bis 5 Gewichtsprozent der Komponente c) enthält.

**12.** Mittel nach einem der Ansprüche 1 bis 11, dadurch gekennzeichnet, daß es 0,1 bis 15 Gewichtsprozent eines nichtionischen Polymers enthält.

**13.** Mittel nach einem der Ansprüche 1 bis 12, dadurch gekennzeichnet, daß es 0,1 bis 15 Gewichtsprozent eines anionischen Polymers enthält.

**Claims**

**1.** Aqueous hair-setting agent in the form of a viscous, sprayable gel with a viscosity of from 200 - 10,000 mPa•s, measured with a Haake VT 501 viscosity meter at 25°C and with a SV-DIN shear rate of 12.9 s$^{-1}$, line B, position 5, with a content of

    a) from 0.1 to 50 per cent by weight of at least one disaccharide or of a mixture of from 2 to 37 per cent by weight of at least one disaccharide and from 63 to 98 per cent by weight of at least one oligosaccharide,

    b) from 0.05 to 30 per cent by weight of at least one cationic polymer,

    c) from 0.05 to 10 per cent by weight of at least one thickener,
    which

    d) is free of monovalent aliphatic alcohols with from 1 to 4 carbon atoms,
    and

    e) contains no inorganic salts of alkali metals or inorganic salts of bivalent or trivalent metals.

**2.** Agent according to Claim 1, characterized in that it is free of organic solvents.

**3.** Agent according to Claim 1, characterized in that it contains mixtures of glucose disaccharides and glucose oligosaccharides as component a).

**4.** Agent according to Claim 3, characterized in that it contains maltose as component a).

**5.** Agent according to any one of Claims 1 to 4, characterized in that it contains from 1 to 25 per cent by weight of component a).

**6.** Agent according to any one of Claims 1 to 5, characterized in that it contains, as component b), a polyvinylpyrrolidone/dimethylaminoethyl methacrylate polymer, a polyvinylpyrrolidone/imidazolimine methochloride copolymer, a dimethyldiallylammonium chloride/sodium acrylate/acrylamide terpolymer, a vinylpyrrolidone/dimethylaminoethyl methacrylate/vinyl caprolactam terpolymer, a quaternised ammonium salt or hydroxyethyl cellulose and a trimethylammonium-substituted epoxy resin, a vinylpyrrolidone/methacrylamidopropyltrimethylammonium copolymer or a di-quaternary polydimethyl siloxane.

**7.** Agent according to any one of Claims 1 to 6, characterized in that it contains from 0.05 to 10 per cent by weight of component b).

**8.** Agent according to any one of Claims 1 to 7, characterized in that it contains, as component c), a homopolymer of acrylic acid, an acrylic acid/acrylamide copolymer, or a sclerotium gum.

**9.** Agent according to Claim 8, characterized in that it contains an acrylic acid homopolymer with an average molecular weight of 4,000,000.

**10.** Agent according to Claim 8, characterized in that it contains an acrylic acid homopolymer with an average molecular weight of from 2,000,000 to 6,000,000.

**11.** Agent according to any one of Claims 1 to 10, characterized in that it contains from 0.1 to 5 per cent by weight of component c).

**12.** Agent according to any one of Claims 1 to 11, characterized in that it contains from 0.1 to 15 per cent by weight of a non-ionic polymer.

**13.** Agent according to any one of Claims 1 to 12, characterized in that it contains from 0.1 to 15 per cent by weight of an anionic polymer.

**Revendications**

1. Agent aqueux pour la fixation des cheveux sous forme d'un gel pulvérisable avec une viscosité de 200-10.000 mPa.s, mesurée avec un viscomètre Haake VT 501 à 25°C et un gradient de cisaillement de 12,9 s$^{-1}$, série B, position 5, contenant:

   a) de 0,1 à 50% en poids d'au moins un disaccharide ou d'un mélange de 2 à 37% en poids d'au moins un disaccharide et 63 à 98% en poids d'un oligosaccharide,

   b) de 0,05 à 30% en poids d'au moins un polymère cationique,

   c) de 0,05 à 10% en poids d'un épaississant, qui

   d) est exempt d'alcool aliphatique monovalent contenant de 1 à 4 atomes de carbone, et

   e) ne contient aucun sel minéral de métaux alcalins ou aucun sel minéral de métaux bi- ou trivalents.

2. Agent selon la revendication 1, caractérisé en ce qu'il est exempt de solvants organiques.

3. Agent selon la revendication 1, caractérisé en ce qu'il contient comme composants (a) des mélanges de gluco-disaccharides et de gluco-oligosaccharides.

4. Agent selon la revendication 3, caractérisé en ce qu'il contient du maltose comme composant (a)

5. Agent selon l'une des revendications 1 à 4, caractérisé en ce qu'il contient de 1 à 25% en poids du composant (a).

6. Agent selon l'une des revendications 1 à 5, caractérisé en ce qu'il contient comme composant (b) un polymère de polyvinylpyrrolidone/diméthylaminoéthyl-méthacrylate, un copolymère de polyvinylpyrrolidone/ méthachlorure d'imidazolimine, un terpolymère de chlorure de diméthyldiallylammonium/acrylate de sodium/acrylamide, un terpolymère de vinylpyrrolidone/méthacrylate de diméthylaminoéthyl/vinyl-caprolactame, un sel d'ammonium quaternisé ou de l'hydroxyéthylcellulose, et un époxide à substitution triméthylammonium, un copolymère de vinylpyrrolidone/-méthacrylamidopropyltriméthylammonium ou un polydiméthylsiloxane diquaternaire.

7. Agent selon l'une des revendications 1 à 6, caractérisé en ce qu'il contient de 0,05 à 10% en poids du composant (b).

8. Agent selon l'une des revendications 1 à 7, caractérisé en ce qu'il contient comme composant (c) un homopolymère de l'acide acrylique, un copolymère acide acrylique/acrylamide, ou une gomme de sclerotium.

9. Agent selon la revendication 8, caractérisé en ce qu'il contient un homopolymère de l'acide acrylique d'un poids moléculaire moyen de 4 000 000.

10. Agent selon la revendication 8, caractérisé en ce qu'il contient un homopolymère de l'acide acrylique d'un poids moléculaire moyen allant de 2 000 000 à 6 000 000.

11. Agent selon l'une des revendications 1 à 10, caractérisé en ce qu'il contient de 0,1 à 5% en poids de composant (c)

12. Agent selon l'une des revendications 1 à 11, caractérisé en ce qu'il contient de 0,1 à 15% en poids d'un polymère non ionique.

13. Agent selon l'une des revendications 1 à 12, caractérisé en ce qu'il contient de 0,1 à 15% en poids d'un polymère anionique.